Europäisches Patentamt

European Patent Office

Office européen des brevets

(11) Numéro de publication: **0 133 070**
**A2**

## (12) DEMANDE DE BREVET EUROPEEN

(21) Numéro de dépôt: **84401356.5**

(22) Date de dépôt: **26.06.84**

(51) Int. Cl.⁴: **C 12 N 9/64**
**A 61 K 37/54**

(30) Priorité: **29.06.83 FR 8310736**

(43) Date de publication de la demande:
**13.02.85 Bulletin 85/7**

(84) Etats contractants désignés:
**AT BE CH DE FR GB IT LI LU NL SE**

(71) Demandeur: **CHOAY S.A.**
**48, Avenue Théophile-Gautier**
**F-75782 Paris Cédex 16(FR)**

(71) Demandeur: **GENEFUSION (S.A.R.L.)**
**48 bis, rue des Belles Feuilles**
**F-75116 Paris(FR)**

(72) Inventeur: **Brouty Boye, Gérard**
**361, rue Lecourbe**
**F-75015 Paris(FR)**

(72) Inventeur: **Maman, Michel**
**2, rue Carves**
**F-92120 Montrouge(FR)**

(72) Inventeur: **Choay, Patrick**
**7, Cour Jasmin**
**F-75016 Paris(FR)**

(72) Inventeur: **Darmon, Michel**
**Verger de Valconstance 300, chemin de la Suquette**
**F-06600 Antibes(FR)**

(74) Mandataire: **Ores, Irène et al,**
**CABINET ORES 6, Avenue de Messine**
**F-75008 Paris(FR)**

(54) **Procédé de production d'un activateur tissulaire du plasminogène et activateur obtenu par ce procédé.**

(57) La présente invention est relative à un procédé de production d'un activateur tissulaire du plasminogène.

Ce procédè se caractérise par la mise en culture et l'incubation d'une catégorie particulière de cellules, à savoir des cellules de tissus embryonnaires, et plus particulièrement des cellules de poumon d'embryon humain dans des conditions déterminées, c'est-à-dire dans un milieu de culture approprié et en présence d'un stimulant comme la concanavaline A et d'inhibiteurs de protéases comme l'aprotinine et/ou la benzamidine, la séparation du surnageant contenant l'activateur tissulaire du plasminogène, l'isolement de ce dernier par purification et éventuellement sa concentration.

EP 0 133 070 A2

-1-

La présente invention est relative à un nouveau procédé de production d'un activateur tissulaire du plasminogène et à l'activateur tissulaire du plasminogène obtenu par ce procédé.

Les activateurs du plasminogène sont des enzymes qui catalalysent la transformation du plasminogène en plasmine. La plasmine ainsi formée peut dégrader la fibrine si les inhibiteurs du sang circulant ne la neutralisent pas. Comme on le sait, les caillots de fibrine qui se forment dans les thromboses résultent de la transformation du fibrinogène présent dans le sang circulant, en fibrine, sous l'action d'une enzyme spécifique, la thrombine. Alors que le sang normal contient un activateur du plasminogène susceptible, en principe, d'induire la dégradation des caillots de fibrine et de les éliminer, il arrive, en fait, que l'activateur du plasminogène soit présent à une concentration insuffisante dans le sang et qu'il faille recourir à une source exogène d'agents favorisant la thrombolyse pour éliminer les caillots intravasculaires.

C'est la raison pour laquelle les recherches effectuées sur les enzymes participant à la fibrinolyse sont très poussées car elles présentent un intérêt primordial pour le développement de médicaments thrombolytiques particulièrement bien adaptés au traitement de la pathologie thromboembolique chez l'homme, et notamment de troubles vasculaires thrombotiques occlusifs qui sont actuellement la cause principale de décès en Europe Occidentale et en Amérique du Nord.

Actuellement, on utilise en thérapeutique principalement l'urokinase qui est un activateur du plasminogène isolé de l'urine humaine et de cellules de reins humains en culture, et la streptokinase qui est un activateur du plasminogène extrait de streptocoques. Toutefois ces activateurs du plasminogène diffèrent de l'activateur tissulaire du plasminogène par leur absence d'affinité spécifique pour la fibrine : en fait la plasmine libérée par les activateurs de

0133070

-2-

plasminogène agit sans discrimination sur le fibrinogène et sur la fibrine en sorte que la lyse d'un caillot n'est obtenue qu'au prix d'une réduction importante du fibrinogène circulant, avec tous les risques d'effets secondaires qu'implique une telle réduction (fibrinogènolyse et hémorragies internes).

C'est la raison pour laquelle les recherches se sont orientées vers l'obtention d'activateurs du plasminogène présentant une structure du type activateur tissulaire du plasminogène du sang, présentant une affinité spécifique pour la fibrine, donc ne détruisant pas le fibrinogène circulant.

Alors que l'urokinase n'est pas absorbée dans les conditions physiologiques sur la fibrine, les activateurs du plasminogène obtenus à partir de tissus d'organes (notamment coeur de porc, ovaires de truie pleine) sont fixés spécifiquement sur la fibrine, ce qui a une incidence physiologique importante sur la fibrinolyse /cf. THORSEN et Alia, THROMBOS. DIATHES. HAEMORRH. (STUTTG.), 1972, 28, 65-747. A partir des travaux de THORSEN et Alia, on a inventorié les tissus propres à fournir de l'activateur du plasminogène présentant une affinité spécifique pour la fibrine : RIJKEN et Alia /Biochemica et Biophysica Acta, 580 (1979) 140-1537 ont obtenu à partir de tissu utérin humain, un activateur tissulaire du plasminogène de poids moléculaire 69 000, avec deux fractions réduites, de poids moléculaires respectivement de 31 000 et de 38 000 et d'activité spécifique variant de 16 000 à 60 000 UI/mg de protéine selon le test mis en oeuvre ; ALLEN CELL BIOLOGY INTERNATIONAL REPORTS, Vol. 4, N° 8, Août 1980 a obtenu un activateur tissulaire de plasminogène à partir de tissu vasculaire humain. D'autres Auteurs ont obtenu de l'activateur de plasminogène à partir de surnageants de cultures de cellules épithéliales de tissus normaux humains ou animaux (cobaye) /VETTERLEIN et Alia, THE JOURNAL OF BIOLOGICAL CHEMISTRY, Vol. 254, N° 3, 10 Février 1979, p 575-578 ; ATKINSON et Alia, THE LANCET, 17 Juillet 1982, p 132-133/, les premiers rapportant l'existence d'un activateur tissulaire de plasminogène de poids moléculaire 73 000 dont l'activité spécifique n'a cependant pas été indiquée.

Il est également connu par le Brevet français n° 2 454 809 au nom d'ASAHI KASEI K.K., de produire un activateur de plasminogène qui n'est pas un activateur tissulaire, à partir de lignées de cellules de reins ou de poumons humains adultes, l'activateur de plasminogène obtenu présentant un poids moléculaire de l'ordre de 45 000 à 68 000, ce qui, compte-tenu de la matière première mise en oeuvre et du poids moléculaire du produit obtenu, laisse à penser que ce dernier devrait être de l'urokinase type II.

Par ailleurs, à la suite de la constatation par divers Auteurs du fait que différents types de tissus tumoraux humains contiennent des concentrations d'activateur de plasminogène plus élevées que les tissus normaux, des activateurs de plasminogène ont été recueillis dans le surnageant de cultures de cellules de mélanomes et d'autres néoplasmes humains ou animaux /cf. notamment WILSON et Alia, CANCER RESEARCH, 40, 933-938, Mars 1980 ; DANO et REICH, THE JOURNAL OF EXPERIMENTAL MEDICINE, Vol. 147, 1978 ; DANO et Alia, BIOCHIMICA et BIOPHYSICA ACTA 613 (1980) 542-555 et Demande de Brevet Européen 0041766 LEUVEN RESEARCH AND DEVELOPMENT V.Z.W./. Il a toutefois été établi /cf. MARQUARDT et Alia, THE JOURNAL OF BIOLOGICAL CHEMISTRY, Vol. 257, N° 9, 10 Mai 1982, p 5220-5225/ que les lignées obtenues par culture de cellules de mélanome métastasique humain peuvent libérer dans le surnageant des cultures, un facteur dit "Human transforming growth factor" qui mis en présence de fibroblastes normaux in vitro leur confère le phénotype transformé des cellules tumorales d'origine. Une telle constatation conduit à renoncer à utiliser comme sources d'activateur de plasminogène, des cultures de tissus tumoraux.

-4-

Il a été proposé, d'autre part, de stimuler la production d'activateur de plasminogène d'une culture de cellules épithéliales de rein de porc par addition de doses subtoxiques de concanavaline A à de telles cultures [cf. MO-CHAN, BIOCHIMICA et BIOPHYSICA ACTA 588 (1979) 273-278].

Enfin, il y a lieu de noter que la Demande de Brevet Européen 0041766 déjà citée plus haut, qui décrit la production d'activateur de plasminogène isolé du surnageant de culture de cellules de mélanome humain, mentionne que la culture, de même que les étapes de purification du surnageant de la culture, peuvent être éventuellement réalisées en présence d'aprotinine pour éviter la dégradation de la molécule d'activateur pendant ces étapes du procédé.

L'utilisation d'inhibiteurs de protéases constitués par l'aprotinine et l'acide 6-aminohexanoïque dans le processus d'obtention d'activateur de plasminogène isolé de tissu délipidé normal de coeur de porc, par purification par adsorption d'affinité sur la fibrine, est également décrite dans Chemical Abstracts, Vol. 98, 1983, p. 199, 2151x ; toutefois, les expérimentations menées par les Demanderesses leur ont permis de mettre en évidence que ces inhibiteurs de protéases ne permettent pas d'obtenir de l'activateur tissulaire du plasminogène.

Des Articles récents (GRONOW et Al., "Trends in Biotechnology", Vol. 1, N° 1, 1983, p. 26-29, et KADOURI et Al., BIOTECHNOLOGY, Juin 1983, p. 354-358) ont discuté de la possibilité de produire de l'activateur de plasminogène à partir de cellules de poumons d'embryons humains, mais le premier de ces Articles a abouti à la conclusion que les cellules de poumons d'embryons humains ne produisent pas d'activateur tissulaire du plasminogène, tandis que les indications contenues dans le second de ces Articles sont telles qu'elles semblent concerner l'urokinase.

Jusqu'à présent, des rendements satisfaisants en activateur tissulaire du plasminogène n'avaient pu être obtenus qu'en mettant en oeuvre des cellules transformées et/ou tumorales, avec tous les inconvénients précités qui en découlent.

La présente invention a pour but de pourvoir à un procédé de production spécifique d'un activateur tissulaire de plasminogène, qui répond mieux aux nécessités de la pratique que les procédés visant au même but antérieurement connus, notamment en ce qu'il utilise comme source d'activateur de plasminogène, des cellules de tissus non tumorales, aisément disponibles en quantités importantes, à croissance relativement rapide lorsqu'elles sont mises en culture, produisant un activateur présentant une affinité spécifique élevée pour la fibrine, et en ce qu'il permet une stimulation de la production de l'activateur tissulaire de plasminogène, avec un haut rendement en activateur par rapport au nombre de cellules initialement mis en oeuvre et bien supérieur à ceux obtenus à ce jour, l'activateur obtenu étant constitué pratiquement en totalité d'activateur tissulaire à affinité pour la fibrine, et ce dans des conditions faciles à mettre en oeuvre au niveau industriel.

La présente invention a pour objet un procédé de production d'un activateur tissulaire de plasminogène à partir de cultures de tissus non tumoraux, caractérisé par : - la mise en culture et l'incubation d'une catégorie particulière de cellules, à savoir des cellules de tissus embryonnaires, et plus particulièrement des cellules de poumon d'embryon humain, dans des conditions données, c'est-à-dire dans un milieu de culture approprié et en présence d'un stimulant comme la concanavaline A et d'inhibiteurs de protéases comme l'aprotinine et/ou la benzamidine ; - la séparation du surnageant contenant l'activateur tissulaire du plasminogène, - l'isolement de ce dernier par purification et éventuellement sa concentration.

Selon un mode de mise en oeuvre préféré du procédé conforme à la présente invention, la mise en culture et l'incubation des cellules de poumons d'embryon humain sont réalisées en présence d'un système stimulant comprenant de la Concanavaline A et des ions Calcium, et d'un inhibiteur de protéases tel que l'aprotinine et/ou la benzamidine.

Un système stimulant comprenant à la fois la Concanavaline A et des ions Calcium a un effet moins traumatisant sur les cellules traitées ; ce système permet en outre d'obtenir de façon privilégiée l'activateur tissulaire présentant une affinité pour la fibrine.

Selon une modalité avantageuse de ce mode de mise en oeuvre, les ions Calcium sont présents à raison de 5-6 mM.

Selon encore un autre mode de mise en oeuvre préféré du procédé conforme à l'invention, les cellules embryonnaires de poumon sont mises en culture dans un milieu de culture approprié contenant un adjuvant de culture tel que du sérum de veau foetal ou du sérum de cheval ou un adjuvant équivalent éventuellement en présence d'une matrice extracellulaire ou de constituants purifiés de nature extracellulaire, puis sont incubées dans le même milieu de culture que ci-dessus, mais dépourvu de sérum de veau foetal et additionné d'insuline, de transferrine et de sélénium, d'un stimulant comme la concanavaline A et d'un inhibiteur de protéases comme l'aprotinine ou la benzamidine.

Selon un autre mode de mise en oeuvre préféré du procédé conforme à l'invention, la concanavaline A est présente dans le milieu de culture à une concentration optimale d'environ 5 à 20 μg/ml et l'aprotinine ou inhibiteur analogue est présente dans le milieu de culture à une concentration optimale équivalant à environ 20 à 50 KIU/ml d'aprotinine.

Par ailleurs, alors que normalement des cellules traitées par la concanavaline A sont très traumatisées, on a pu déterminer, conformément à l'invention, des conditions qui permettent de restaurer la capacité des cellules traitées à induire à nouveau la production d'activateur tissulaire de plasminogène.

Selon un mode de mise en oeuvre préféré du procédé conforme à l'invention, ces conditions consistent en ce que, pour permettre une réutilisation des cellules traitées par la concanavaline A au cours d'une première opération d'induction de production d'activateur tissulaire de plasminogène, pour au moins une deuxième opération de production d'activateur tissulaire de plasminogène, on réincube la culture de cellules traumatisées par la concanavaline A, après séparation du surnageant, dans un milieu de culture approprié contenant du sérum de veau foetal, pendant une durée suffisante pour restaurer les cellules dans leur intégrité, puis on renouvelle le processus d'induction par la concanavaline A en présence d'aprotinine, dans le milieu de culture précité dépourvu de sérum de veau foetal et contenant de l'insuline, de la transferrine et du sélénium, et on sépare le nouveau surnageant qui contient l'activateur tissulaire de plasminogène, les restauration et réinduction des cellules pouvant être opérées à plusieurs reprises successives, au moins trois fois.

Ce processus présente un grand intérêt pour la production industrielle du produit de l'invention car il évite des opérations longues, fastidieuses et onéreuses.

Selon un autre mode de mise en oeuvre préféré du procédé conforme à l'invention, le surnageant de culture est additionné d'un agent tensio-actif approprié propre à empêcher l'adsorption de l'activateur présent dans le surnageant, sur les parois des récipients de traitement.

Selon encore un autre mode de mise en oeuvre préféré du procédé conforme à l'invention, le surnageant de culture séparé de la culture est purifié par chromatographie d'affinité sur une préparation de fibrine déposée sur une matrice solide adsorbante, en terre de diatomées, du type de la Célite, qui adsorbe sélectivement l'activateur tissulaire du plasminogène à haute affinité, lequel est élué de la colonne adsorbante à l'aide d'une solution tampon appropriée contenant de l'arginine.

Selon une modalité avantageuse de ce mode de mise en oeuvre, l'activateur tissulaire de plasminogène purifié sur fibrine/Célite est concentré par ultrafiltration, puis soumis à une purification complémentaire par gel-filtration.

Conformément à l'invention, cette purification complémentaire par gel-filtration est réalisée sur une colonne d'agarose (du type "SEPHADEX") ou d'acrylamide-agarose (du type de l'"ULTROGEL").

Selon une autre modalité avantageuse de ce mode de mise en oeuvre, le produit ainsi obtenu peut être concentré sur membrane calibrée par ultrafiltration et ensuite dialysé contre un tampon approprié.

Pour la mise en oeuvre du procédé conforme à l'invention, l'on procède comme suit :

Mise en culture et établissement de lignées de cellules de poumon embryonnaire humain.

Les poumons sont prélevés sur des embryons humains de 3 à 4 mois et découpés en fragments de 1 à 2 mm$^3$ environ à l'aide de scalpels. Les fragments sont rincés dans du milieu de culture sans sérum puis disposés dans des récipients en plastique contenant du milieu de culture additionné de 10 % de sérum de veau nouveau-né. Huit à dix jours plus tard, les cellules issues de ces fragments ont atteint la confluence. Les cellules sont sub-cultivées à raison de trois flacons pour un flacon après avoir été décollées par une solution de trypsine. Pour la production d'activateurs du plasminogène les lignées ont été utilisées entre la dixième et la trentième sub-culture.

Caractérisation de ces lignées

L'analyse chromosomique a été effectuée par la méthode de Hsu dans Tissue Culture, Methods and Applications, Kruse P.F. and Patterson M.K. (Eds) Academic Press Inc., New-York 1973, p. 764. Le caryotype est diploïde avec 4c chromosomes.

L'absence de mycoplasmes a été contrôlée à l'aide de la méthode de coloration par le fluorochrome Hoechst 33258 dans Laboratory Techniques in Biochemistry and Molecular Biology, Vol. 8, T.S. Work and R.H. Burdon (Eds) Elsevier/ North-Holland 1980, p. 132.

L'absence de production de "Human Transforming Growth Factor" a été vérifiée à l'aide de la méthode décrite par J.E. de Larco et J.G. Todaro dans Proc. Natl. Acad. Sci. U.S.A., Vol. 75, n° 8, p. 4001-4005, Août 1978.

### 1ère variante du procédé de production d'activateur tissulaire du plasminogène.

Les cellules de poumon d'embryon humain sont ensemencées dans des récipients en plastique à une concentration minimale de $10^4$ cellules/cm$^2$ de préférence, permettant d'obtenir la confluence en 4 à 5 jours, dans un milieu de culture DMF composé de trois parties de milieu minimum essentiel de Eagle modifié par DULBECCO et d'une partie de milieu de Ham F 12, additionné de 10 % de sérum embryonnaire de veau (SVF) préalablement inactivé par chauffage pendant 30 minutes à 56°C, d'Hépès 10 mM et de gentamycine 40 µg/ml.

Une fois la confluence cellulaire obtenue, les cellules sont maintenues pendant 3 jours supplémentaires dans le milieu de culture susdit, puis le milieu de culture est éliminé et les cellules sont rincées 2 à 3 fois avec du tampon phosphate salin. Elles sont ensuite mises en incubation dans un milieu de culture DMF dépourvu de SVF et contenant de l'insuline 10 µg/ml, de la transferrine 10 µg/ml, du sélénium $2,5.10^{-8}$M, de la concanavaline A 20 µg/ml et de l'aprotinine 20 à 50 KIU/ml. La durée d'incubation à 37°C $\pm$ 0,5°C est de 16 à 18 heures.

Le surnageant de culture est collecté, centrifugé, puis additionné d'un agent tensio-actif tel que Tween 80 ou TRITON X 100 à la dose de 0,01 % (v/v). Ce produit brut est conservé à - 25°C sans perdre son activité spécifique qui est de 20-40 unités CTA/ml mesurée par caséinolyse radiale selon la méthode de SAKSELA, Anal, Biochem. (1981) 111, 276-282, ou sur une plaque de fibrine selon la méthode de PLOUGH et Alia, BIOCHIM. BIOPHYS. ACTA (1957) 24, 278-282 ou selon la méthode de BEATTIE et Alia Br. J. Haematol. (1976) 32, 135-143.

La concanavaline A ayant une action traumatisante sur les cellules en culture, celles-ci ne peuvent pas être réutilisées telles quelles pour une nouvelle opération de production d'activateur tissulaire de plasminogène et doivent à nouveau être incubées pendant 7 à 10 jours dans du milieu DMF contenant 10 % de SVF. Une fois que les cellules ont de nouveau atteint la confluence, le cycle d'induction par la concanavaline A en présence d'aprotinine est recommencé comme décrit plus haut.

Le nombre d'inductions qui peuvent être réalisées sur une même culture peut atteindre 3 inductions avec un rendement semblable.

2ème variante du procédé de production d'activateur tissulaire du plasminogène.

Les cellules de poumon d'embryon humain sont cultivées jusqu'à confluence, comme décrit dans la première variante, dans un milieu de culture DMF-Ham F 12, un volume pour un volume, contenant de l'Hépès 15 mM et du bicarbonate de sodium 1,4 g/l, pH 7,4, dans des flacons FALCON présentant une grande surface de culture (175 cm$^2$), contenant 60 ml dudit milieu.

Une fois la confluence obtenue, la culture est poursuivie comme décrit dans la première variante, à ceci près que le milieu de culture contenant l'insuline, la transferrine et le sélénium, comme indiqué plus haut, contient en outre de la concanavaline A 20 µg/ml, un sel de calcium, de préférence du chlorure de calcium, 5-6 mM, et de l'aprotinine 20-50 KIU/ml.

Comme la présence d'ions Calcium réduit considérablement l'effet traumatisant de la Concanavaline A sur les cellules cultivées, le nombre d'inductions qui peuvent être réalisées sur une même culture peut dépasser 5 inductions

0133070

-11-

avec un rendement semblable.

En outre, la présence d'ions Calcium dans le milieu de culture permet de doubler le volume de milieu de culture présent dans chaque flacon sans qu'il se produise de phénomènes de limitation de la production d'activateur, le doublement du volume de milieu de culture par flacon permettant un doublement de la production d'activateur tissulaire du plasminogène, par flacon.

Purification

Quelle que soit la variante mise en oeuvre, le surnageant récolté à la suite de l'opération, ou de chaque opération, d'induction est purifié par chromatographie d'affinité sur fibrine/Célite selon la méthode décrite dans HUSAIN et Alia, PROC. NAT. ACAD. SCI. USA, Vol. 78, N° 7, p 4265-4269, Juillet 1981.

L'activateur de plasminogène est recueilli par élution à l'aide d'un tampon phosphate-arginine (ou lysine) contenant de l'aprotinine.

L'activité totale, déterminée sur substrat chromogène S 2288 de la Société KABI, ramenée à 1 litre de surnageant de culture est de 2,5 µkatals.

L'activité totale exprimée par rapport à l'urokinase sur le substrat S 2288 et ramenée à 1 litre de surnageant de culture est comparable à environ 2 500 000 UI CTA d'urokinase (CTA = Committee Thrombolytic Activity).

La mise au point par les Inventeurs d'une méthode de dosage ELISA très spécifique qui utilise un anticorps-antiactivateur tissulaire du plasminogène qui ne croise pas avec l'urokinase, permet d'attribuer avec précision l'activité, exprimée en Unités CTA, qui est spécifique à l'activateur tissulaire du plasminogène (TPA) et de distinguer cette activité d'une éventuelle activité urokinase, tandis que l'activité totale du produit est dosée sur fibrine.

Cette purification qui isole l'activateur tissulaire de plasminogène peut être complétée par chromatographie de

-12-

gel-filtration sur SEPHADEX G 150 ou ULTROGEL AcA 44 et permet d'obtenir une activité spécifique supérieure à 30 000 UI CTA/mg$^{-1}$ de protéines (dosage effectué sur plaque de fibrine).

L'activateur purifié obtenu est ensuite concentré par ultrafiltration sur membrane n'absorbant pratiquement pas les protéines (AMICON PM 10 ou Polysulfone Millipore ou fibres creuses Amicon), cette opération de concentration étant éventuellement complétée par passage sur une colonne de SEPHADEX G 150 ou d'ULTROGEL AcA 44.

A chaque étape, le dosage de l'activateur tissulaire de plasminogène est effectué d'une part sur fibrine et d'autre part sur caséine.

Après purification et concentration, le volume du concentré obtenu à partir de 800 ml de surnageant, est de 51 ml.

La teneur en protéines est inférieure à 10 µg par ml.

L'activité sur plaque de fibrine est de 350 UI CTA par ml, soit une activité spécifique supérieure à 35 000 UI CTA par mg de protéines.

L'activité sur substrat chromogène S 2288 est de 11,25 µkatals par litre.

L'activité sur substrat chromogène S 2288 par rapport à l'urokinase est de 12 000 UI par ml, soit une activité spécifique supérieure à 1 200 000 UI par mg de protéines.

Le produit purifié mis en présence d'anticorps igG anti-urokinase n'est pas inhibé, ce qui démontre qu'on est bien en présence d'activateur tissulaire et non d'urokinase ou d'une enzyme similaire à cette dernière.

La lyophilisation du produit purifié ne semble pas altérer son activité.

Les poids moléculaires des activateurs obtenus conformément à l'invention ont été mesurés par zymographie sur gel de polyacrylamide par comparaison des bandes fibrinolytiques obtenues sur le gel, avec des produits de référence déposés en même temps sur le gel par électrophorèse.

Cette technique a mis en évidence la présence de plusieurs espèces moléculaires de poids moléculaires compris entre 35 000 et 105 000, la majeure partie des espèces moléculaires étant comprise entre PM 50 000 et PM 100 000, avec trois taches principales à : 78 000 66 000 et 55 000,

la tache majoritaire correspondant à un poids moléculaire de 55 000.

L'activateur tissulaire de plasminogène obtenu conformément à la présente invention est en outre caractérisé par :

- son affinité pour la fibrine ;
- son activité plasminogène-dépendante sur la fibrine ;
- ses poids moléculaires, déterminés par SDS Page et par zymographie et éventuellement par gel-filtration ;
- sa très faible activité sur substrat S 2244 spécifique de l'urokinase ;
- l'absence d'inhibition par l'anticorps spécifique anti-urokinase ;
- son activité sur plaque de fibrine ;
- son activité sur substrat S 2288.

Les deux propriétés énoncées en premier peuvent se démontrer par le test de E. VAIREL (cf. "Progress in chemical fibrinolysis and Thrombolysis", Vol. 1, p 271-280, Ed. par J.F. DAVIDSON, M.M. SAMAMA, et P.C. DESNOYERS, Raven Press, New-York, 1975).

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

0133070

-14-

Il doit être bien entendu, toutefois, que ces exemples de mise en oeuvre, sont donnés uniquement à titre d'illustration de l'objet de l'invention, dont ils ne constituent en aucune manière une limitation.

EXEMPLES

Exemple 1

Les cellules embryonnaires de poumon humain sont ensemencées dans des récipients en plastique de 150 cm$^2$ de surface à une concentration de $10^4$ cellules par cm$^2$ dans 30 ml de milieu DMF composé de trois parties de milieu minimum essentiel de Eagle modifié par Dulbecco (Dulbecco R., Freeman G., Virology 8, 396, 1959) et d'une partie de milieu de Ham F 12 (Ham R.G., Proc. Nat. Acad. Sci. US, 53, 288, 1965). Il est additionné de 10 % de sérum embryonnaire de veau inactivé pendant 30 minutes à 56°C, d'Hépès 10 mM, et de gentamycine 40 µg/ml.

Une fois la confluence cellulaire obtenue, les cellules sont conservées trois jours supplémentaires dans cet état.

Le milieu de culture est alors éliminé et les cellules sont rincées 3 fois avec du tampon phosphate salin.

Elles sont mises en incubation dans du milieu DMF (0,7 ml/$10^5$ cellules) contenant de l'insuline 10 µg/ml, de la transferrine 10 µg/ml, du sélénium $2,5.10^{-8}$M, de l'aprotinine 20 KIU/ml. La durée d'incubation est de 18 heures à une température comprise entre 36,5 et 37,5°C.

Dans ces conditions, le titre du produit brut obtenu est de 1 uCTA/ml.

Exemple 2

Les cellules sont cultivées dans les mêmes conditions que dans l'Exemple 1. Après trois lavages avec du tampon phosphate salin, elles sont mises en incubation dans du milieu DMF (0,7 ml/$10^5$ cellules) contenant de l'insuline 10 µg /ml, de la transferrine 10 µg/ml, du sélénium $2,5.10^{-8}$M, de l'aprotinine 20 KIU/ml, de la concanavaline A 20 µg/ml.

-15-

La durée d'incubation est de 18 heures à une température comprise entre 36,5 et 37,5°C.

Dans ces conditions, le titre du produit brut obtenu est de 30 uCTA/ml.

Exemple 3

Les cellules sont ensemencées dans les mêmes conditions que dans l'exemple 1. Une fois la confluence atteinte, les cellules sont rincées 3 fois avec du tampon phosphate salin. Elles sont ensuite mises en incubation dans du milieu DMF (0,7 ml/$10^5$ cellules) contenant de l'insuline 10 µg/ml, de la transferrine 10 µg/ml, du sélénium $2,5.10^{-8}$M, de l'aprotinine 20 KIU/ml, de la concanavaline A 20 µg/ml. La durée d'incubation est de 18 heures à une température comprise entre 36,5 et 37,5°C. Au bout de ce temps, la première récolte est effectuée.

Les cellules sont alors remises en incubation dans du milieu DMF additionné de 10 % de sérum embryonnaire de veau pendant 7 à 10 jours pour leur permettre de réatteindre la confluence après l'effet traumatisant de la concanavaline A.

Une fois la confluence atteinte, le processus d'induction par la concanavaline A en présence d'aprotinine est renouvelé une deuxième fois, ainsi que décrit dans l'exemple 2.

Une deuxième récolte est alors effectuée.

Les cellules sont, de nouveau, mises en incubation dans du milieu DMF additionné de 10 % de sérum embryonnaire de veau pendant 7 à 10 jours comme expliqué précédemment.

Un troisième processus d'induction par la concanavaline A en présence d'aprotinine est alors mis en oeuvre et donne lieu à une troisième récolte.

Les titres obtenus sont les suivants :

        1ère récolte : 30 uCTA/ml

        2ème récolte : 40 uCTA/ml

        3ème récolte : 30 uCTA/ml

Exemple 4

800 ml de surnageant de culture contenant de l'aprotinine à 20 KIU/ml, provenant d'une culture sur un multitray (6000 cm$^2$).

Titre 10 UI CTA/ml (plaque de fibrine) 40 UI CTA/ml (plaque de caséine).

Teneur en protéines : 39 µg/ml.

1. Chromatographie sur fibrine/Célite

Sur une colonne 8 X 18 cm de fibrine/Célite équilibrée en tampon phosphate 50 mM, NaCl 300 mM, aprotinine 10 KIU/ml, EDTA 1 mM, Azide 0,02 % pH 7,4.

On fait passer 800 ml de surnageant de culture.

Elution par le même tampon rendu 0,2 M en arginine et contenant 0,01 % de Tween 80.

On obtient 2 fractions dosées sur plaque de fibrine et substrat chromogène S 2288.

1ère fraction : 900 ml à 2 363 UI/ml = 2 126 700 UI 2ème fraction : 900 ml à 500 UI/ml = 450 000 UI.

(activités obtenues sur substrat chromogène S 2288 par rapport à l'urokinase).

2. Concentration.

Par ultrafiltration sur membrane Millipore, ref. PTGC 0001 cut-off 10 000.

La fraction 1 : volume 50 ml à 34 440 UI/ml = 1 722 000 UI La fraction 2 : volume 16 ml à 11 550 UI/ml =   184 000 UI Taux de protéines :

Fraction 1 concentrée inférieure à 20 µg/ml,

Fraction 2 concentrée inférieure à 20 µg/ml.

La présence d'aprotinine dans les solutions ne permet pas d'envisager un dosage autre que sur le S 2288.

3. Chromatographie sur ULTROGEL AcA 44.

Dépôt de la fraction 1 concentrée. Développement de la chromatographie à l'aide de solution $NH_4HCO_3$ 1 M, Tween 0,01 % pH 7,4 sans aprotinine. Contrôle et dosage sur plaque de fibrine. Volume : 270 ml.

-17-

4. Concentration.

Comme au paragraphe 2 ci-dessus.

Volume du concentré : 78 ml Activité sur S 2288/UK : 12 000 UI/ml UI totales : 936 000 UI.

Activité sur plaque de fibrine : 350 UI CTA/ml Teneur en protéines : inférieure à 10 µg/ml

Activité spécifique sur plaque de fibrine supérieure à 35 000 UI CTA par mg de protéines.

- Test d'activité plasminogène-dépendant : conforme.

- Test sur S 2444 (spécifique urokinase) : négligeable.

- Test d'affinité sur fibrine : conforme.

- Electrophorèse : Zymographie 3 bandes actives : 55 000, 66 000 et 78 000 dont la principale est environ 55 000.

- Pas de réaction avec le sérum anti-urokinase.

5. Lyophilisation.

Le produit est ensuite lyophilisé en présence d'un stabilisant tel qu'albumine ou héparine.

Exemple 5

Les cellules sont ensemencées dans des flacons FALCON de 175 cm$^2$ de surface à une concentration de $10^4$ cellules/cm$^2$ dans 60 ml de milieu DMF-HAM F 12, un volume pour un volume, contenant de l'Hépès 15 mM et du bicarbonate de sodium 1,4 g/l, pH 7,4.

Une fois la confluence cellulaire obtenue, les cellules sont conservées 3 jours supplémentaires en état de confluence, puis le milieu de culture est éliminé et les cellules sont rincées 3 fois avec du tampon phosphate salin.

Elles sont ensuite mises en incubation dans du milieu DMF (0,7 ml/$10^5$ cellules) contenant de l'insuline 10 µg/ml, de la transferrine 10 µg/ml, du sélénium $2,5.10^{-8}$M, de la Concanavaline A 10 µg/ml, du chlorure de calcium 6 mM, de l'aprotinine 20 KIU/ml. La durée d'incubation est de 18 heures à une température comprise entre 36,5 et 37,5°C.

Alors que la quantité d'activateurs du plasminogène produite naturellement par les cellules de poumon d'embryon humain est de 0,4 unité CTA/ml, l'action de stimulation exercée par la concanavaline A augmente le rendement en activateur de 20 à 40 fois et permet, après purification et concentration, d'obtenir un activateur tissulaire d'activité spécifique supérieure à 35 000 UI CTA par mg de protéines, l'addition d'aprotinine permettant une stabilisation des activateurs obtenus, qui en l'absence d'aprotinine seraient très rapidement dégradés. Si l'introduction dans le système d'induction de la production d'activateur tissulaire du plasminogène conforme à l'invention, d'ions calcium ne paraît pas avoir d'incidence sur l'activité CTA totale, par contre elle augmente considérablement l'activité spécifique TPA par rapport à l'activité Urokinase (UK), ainsi que cela ressort très clairement du test dont il est rendu compte ci-après.

En mettant en oeuvre le mode opératoire décrit à l'Exemple 5 ci-dessus, on introduit dans le milieu d'incubation les substances suivantes :

- aucune
- 20 µg/ml de Concanavaline A
- 20 µg/ml de Concanavaline A + 20 KIU/ml d'aprotinine
- 20 µg/ml de Concanavaline A + 20 KIU/ml d'aprotinine
  + 6 mM de CaCl$_2$
- 20 KIU/ml d'aprotinine seule
- 6 mM de CaCl$_2$ seul

L'activité de l'activateur tissulaire du plasminogène conforme à l'invention, obtenu après purification et concentration, est dosée - sur plaque de fibrine, pour l'activité totale
- en système ELISA utilisant un anticorps anti-TPA tel qu'indiqué plus haut, pour le TPA.

0133070

-19-

Les résultats obtenus sont réunis dans le Tableau qui va suivre.

| Additifs en milieu de culture | Unités CTA totale | Unités TPA (en CTA) | Unités UK (en CTA) |
|---|---|---|---|
| 1°) rien | 0-4 | 0 | 0-4 |
| 2°) Concanavaline A 20 µg/ml | 20 | 5 | 15 |
| 3°) Concanavaline A 20 µg/ml + Aprotinine 20 KIU/ml | 19 | 13 | 6 |
| 4°) Concanavaline A 20 µg/ml + Aprotinine 20 KIU/ml + CaCl$_2$ 6 mM | 19-20 | 19 | 0-1 |
| 5°) Aprotinine seule 20 KIU/ml | 0-4 | 0 | 0-4 |
| 6°) CaCl$_2$ seul 6 mM | 0-4 | 0 | 0-4 |

L'inclusion d'ions Calcium dans le système d'induction a pour effet d'augmenter la production d'activateur tissulaire du plasminogène par rapport à l'activité urokinase du produit conforme à l'invention, et par voie de conséquence, d'augmenter l'affinité spécifique du produit conforme à l'invention pour la fibrine et de réduire considérablement, voire d'éliminer, les risques d'effets secondaires liés à l'activité urokinase.

L'on obtient par le procédé conforme à l'invention un activateur tissulaire du plasminogène à haute affinité pour la fibrine, qui ne détruit pas le fibrinogène circulant et peut donc réaliser in vivo la lyse d'un caillot à des doses thérapeutiques considérablement plus faibles (environ 30 fois moins) à celles nécessaires avec l'urokinase, l'activateur tissulaire étant en outre obtenu avec un bon rendement qui permet sa fabrication à l'échelle industrielle dans des conditions économiques satisfaisantes.

L'action d'activation de la lyse d'un caillot exercée par l'activateur tissulaire du plasminogène obtenu conformément à la présente invention, a été testée en la comparant à celle de l'urokinase dans un essai pharmacologique in vitro dont les modalités et les résultats seront décrits ci-après.

Essai pharmacologique in vitro d'activation de la lyse d'un caillot respectivement par l'activateur tissulaire de plasminogène conforme à l'invention et par l'urokinase

Le produit obtenu dans l'exemple 2 et de l'urokinase, ont été soumis respectivement à un test de lyse d'un caillot selon la méthode de E. VAIREL décrite dans l'Ouvrage déjà cité. Le caillot utilisé est un caillot déplaquetté pour assurer une bonne adhérence au tube à essai.

Les étapes de l'expérimentation sont les suivantes :

A. Première expérimentation avec l'urokinase.

    1. Préparation d'un caillot déplaquetté et lavage de ce dernier pour élimination de toute trace de plasminogène.

    2. Passage d'une solution contenant de l'urokinase. On ne constate pas de lyse puisqu'il n'y a pas de plasminogène.

    3. Lavage.

    4. Passage d'une solution de plasminogène purifié. On ne constate pas de lyse. En effet l'urokinase ne se fixe pas sur la fibrine et a été éliminée au cours du lavage précédent.

B. Deuxième expérimentation en utilisant l'activateur tissulaire.

On réalise ensuite une deuxième expérimentation dans les mêmes conditions, en remplaçant l'urokinase de l'étape 2 par une solution d'activateur tissulaire du plasminogène obtenu selon l'Exemple 2. On constate à l'étape 4 une lyse du caillot de fibrine. En effet, l'activateur tissulaire s'est fixé sur la fibrine et active le plasminogène au moment de son passage dans le caillot.

Ainsi qu'on l'a mentionné dans le Préambule, la présence de "Human Transforming Growth Factor" ou "facteur transformant de croissance humain" dans un activateur tissulaire de plasminogène, constitue un obstacle dirimant à son utilisation en tant que médicament thrombolytique ; or l'un des avantages que présente l'activateur tissulaire conforme à l'invention, est de ne pas contenir de "Human Transforming Growth Factor" puisqu'il provient de cellules non transformées. Cette absence de "Human Transforming Growth Factor" a été vérifiée par les Inventeurs à l'aide de la méthode décrite par J.E. DE LARCO et G.J. TODARO dans Proc. Natl. Acad. Sci. U.S.A., Vol. 75, N° 8, p. 4001-4005, Août 1978.

Les résultats de ce contrôle sont les suivants :

. Essai sur le surnageant provenant de cultures de cellules embryonnaires de poumon humain :

    Réponse négative : pas de facteur transformant de croissance.

. Essai sur le produit brut obtenu par action de la concanavaline A en présence d'aprotinine, conformément à l'Exemple 2 :

    Réponse négative : pas de facteur transformant de croissance.

. Essai sur le produit brut obtenu conformément à l'Exemple 3 à la suite du troisième processus d'induction par la concanavaline A en présence d'aprotinine :

    Réponse négative : pas de facteur transformant de croissance.

L'activateur tissulaire de plasminogène à haute affinité pour la fibrine, dépourvu de facteur transformant de croissance et présentant une activité spécifique égale ou supérieure à 35 000 uCTA/mg, obtenu conformément à la présente invention, semble donc bien constituer le médicament thrombolytique de grande valeur, sans action destructrice sur le fibrinogène, dont souhaite pouvoir disposer le corps médical.

-23-

REVENDICATIONS

1°) Procédé de production d'un activateur tissulaire de plasminogène à partir de cultures de tissus non tumoraux, caractérisé par : - la mise en culture et l'incubation d'une catégorie particulière de cellules, à savoir des cellules de tissus embryonnaires, et plus particulièrement des cellules de poumon d'embryon humain dans des conditions déterminées, c'est-à-dire dans un milieu de culture approprié et en présence d'un stimulant comme la concanavaline A et d'inhibiteurs de protéases comme l'aprotinine et/ou la benzamidine ; - la séparation du surnageant contenant l'activateur tissulaire du plasminogène, - l'isolement de ce dernier par purification et éventuellement sa concentration.

2°) Procédé selon la revendication 1, caractérisé en ce que le système stimulant comprend outre la Concanavaline A, des ions Calcium.

3°) Procédé selon l'une quelconque des revendications 1 et 2, caractérisé en ce que les cellules embryonnaires de poumon sont mises en culture dans un milieu approprié contenant un adjuvant de culture tel que le sérum de veau foetal ou du sérum de cheval ou un adjuvant équivalent, éventuellement en présence d'une matrice extracellulaire ou de constituants purifiés de nature extra-cellulaire, puis sont incubées dans le même milieu de culture que ci-dessus, mais dépourvu de sérum de veau foetal et additionné d'insuline, de transferrine et de sélénium, d'un stimulant comme la concanavaline A et éventuellement d'ions Calcium et d'un inhibiteur de protéases comme l'aprotinine ou la benzamidine.

4°) Procédé selon l'une quelconque des revendications 1 à 3, caractérisé en ce que la concanavaline A est présente dans le milieu de culture à une concentration optimale d'environ 5 à 20 µg/ml et l'aprotinine ou inhibiteur analogue est présente dans le milieu de culture à une concentration optimale équivalant à environ 20 à 50 KIU/ml.

5°) Procédé selon la revendication 2, caractérisé en ce que les ions Calcium sont présents à raison de 5-6 mM dans le milieu de culture.

6°) Procédé selon l'une quelconque des revendications 1 à 5, caractérisé en ce que, pour permettre une réutilisation des cellules traitées par la concanavaline A au cours d'une première opération d'induction de production d'activateur tissulaire de plasminogène, pour au moins une deuxième opération de production d'activateur tissulaire de plasminogène, on réincube la culture de cellules traumatisées par la concanavaline A, après séparation du surnageant, dans un milieu de culture approprié contenant du sérum de veau foetal pendant une durée suffisante pour restaurer les cellules dans leur intégrité, puis on renouvelle le processus d'induction par la concanavaline A en présence d'aprotinine, dans le milieu de culture précité dépourvu de sérum de veau foetal et contenant de l'insuline, de la transferrine et du sélénium, et on sépare le nouveau surnageant qui contient l'activateur de plasminogène, les restauration et réinduction des cellules pouvant être opérées à plusieurs reprises, et au moins à trois reprises.

7°) Procédé selon l'une quelconque des revendications 1 à 6, caractérisé en ce que le surnageant de culture est additionné d'un agent tensio-actif approprié propre à empêcher l'adsorption de l'activateur présent dans le surnageant sur les parois des récipients de traitement.

8°) Procédé selon l'une quelconque des revendications 1 à 7, caractérisé en ce que le surnageant de culture séparé de la culture est purifié par chromatographie d'affinité sur une préparation de fibrine déposée sur une matrice solide adsorbante, en terre de diatomées, du type de la Célite, qui adsorbe sélectivement l'activateur tissulaire du plasminogène à haute affinité, lequel est élué de la colonne adsorbante à l'aide d'une solution tampon appropriée contenant de l'arginine ou de la lysine.

9°) Procédé selon la revendication 8, caractérisé en ce que l'activateur de plasminogène purifié sur fibrine/Célite est concentré par ultrafiltration, puis soumis à une purification complémentaire par gel-filtration.

10°) Activateur tissulaire du plasminogène caractérisé en ce qu'il est obtenu par le procédé selon l'une quelconque des revendications 1 à 9.

11°) Activateur tissulaire du plasminogène selon la revendication 10, caractérisé en ce qu'il est dépourvu de facteur transformant de croissance, en ce qu'il se compose d'une pluralité d'espèces moléculaires présentant des poids moléculaires compris entre 35 000 et 105 000 daltons, la majeure partie des espèces moléculaires se situant entre 50 000 et 80 000 daltons avec des espèces majoritaires de poids moléculaires 55 000, 66 000 et 78 000, en ce qu'il ne présente pas d'affinité pour les anticorps anti-urokinase, en ce qu'il est dépourvu d'activité sur les substrats analytiques protidiques spécifiques de l'urokinase, en ce qu'il possède une haute affinité pour la fibrine et présente une activité spécifique égale ou supérieure à 35 000 uCTA/mg.

12°) Composition thérapeutique, caractérisée en ce qu'elle comprend une quantité thérapeutiquement active de l'activateur tissulaire du plasminogène selon l'une quelconque des revendications 10 et 11, associée à un véhicule physiologiquement acceptable.